# EUROPEAN PATENT SPECIFICATION

(11) **EP 0 911 031 B1**
(45) Date of publication and mention of the grant of the patent: **15.12.2004**
(21) Application number: 98203139.5
(22) Date of filing: 17.09.1998
(51) Int. Cl.: A61K 33/24, A61K 31/285, A61K 33/36

(54) **Use of arsenic for preparing a medicament for the treatment of premenstrual syndrome**
Verwendung von Arsen zur Herstellung eines Arzneimittels zur Behandlung vom prämenstruellen Syndrom
Utilisation de l'arsenic dans la préparation d'un médicament pour le traitement du syndrome prémenstruel

(30) Priority: 25.09.1997 IT MI972170
(43) Date of publication of application: 28.04.1999
(73) Proprietor: TARELLO, Walter, I-06061 Castiglione del Lago (Perugia) (IT); Riccieri, Natascia, 06100 Perugia (IT)
(72) Inventor: TARELLO, Walter, I-06061 Castiglione del Lago (Perugia) (IT); Riccieri, Natascia, 06100 Perugia (IT)
(74) Representative: Marchi, Massimo

(56) References cited:
- WO-A-94/21249
- WO-A-95/01789
- WO-A-99/38499
- US-A- 4 755 384

## Description

The present invention relates to the use of organic and inorganic arsenic compounds for preparing a medicament for the treatment of premenstrual syndrome.

Premenstrual syndrome (PMS) is characterized by a number of cyclically recurring clinical symptoms which affect the cognitive, emotional, physical and behavioural capacities of women patients in the final stage of the luteinic phase of the menstrual cycle.

Among the abovementioned physical symptoms, those occurring most regularly are tenderness and swelling of the breasts, nausea, vomiting, abdominal pains, intestinal disturbances, headaches, muscular and articular pains and oedema. Among the emotional and cognitive symptoms, the most common are depression, rapid mood changes, outbursts of anger, irritability and anxiety. Other recurrent symptoms in PMS are: reduced interest in normal daily activities, difficulty in concentrating, tiredness, insomnia or hypersomnia, water retention, increased appetite and hunger pangs. These symptoms are exhibited in different combinations in the final part of the luteinic phase and disappear during the menstrual flow. They can also appear at any time after ovulation and, in general, at any time after menarche.

Furthermore, these symptoms gradually become worse with age, until menopause when PMS ceases.

Unfortunately, there is no specific test for PMS and its diagnosis is arrived at by exclusion. Its aetiology is unknown and the treatments developed to date are symptomatic and give conflicting results.

Examples of such treatments are those which involve the use of: progesterone (US patent 5,093,265), a composition containing ibuprofen/hydrochloro-thiazide (US patent 5,155,105), calcium (US patent 4,946,679), an aqueous iodine-based solution (US patent 5,250,304), melatonin (US patent 4,945,103), herbs such as *Arnica montana, Symphytum officianale* and others (US patent 5,162,037), acetaminophen/caffeine/diuretic ammonia salt/antihistamine (US patent 4,466,960), ascorbic acid and salts thereof (US patent 4,414,212).

Other treatments involve the use of analogues of luteinizing hormone release factor (LHRH), oral contraceptives, oestradiol implants or danazole.

Benzodiazepines such as Alprazolam, Fluoxetine, Buspirone, Nortriptyline and Clomipramine, for example, are used to control the emotional symptoms.

Diuretics have been used to combat the water retention, but there is no specific treatment for the irritability, perhaps because sufficient attention has not yet been devoted to the search for a treatment for this symptom.

Tocoferol and oil of *Mirabilis jalapa* (Evening Primrose Oil) have also been proposed to combat mastalgia, and fenfluoramine has been proposed to combat hunger pangs.

Besides these pharmacological treatments, changing the lifestyle has also been proposed to alleviate certain symptoms. For example, eliminating the consumption of tea and coffee can reduce the irritability and insomnia, while eliminating salt from the diet and taking regular physical exercise can attenuate the retention of fluids.

Lastly, experiments have been conducted on the administration of pyridoxine (vitamin B6) to control symptoms such as depression, irritability and tiredness (Sally K. Severino and Margaret L. Moline, "Premenstrual Syndrome: Identification and Management Drugs", 49(1), 71-82, 1995).

The wide variety and diversity of the treatments proposed to date is a clear indication of the fact that the research is oriented in many directions and that the results obtained are still unsatisfactory.

It has now been found that premenstrual syndrome can be treated advantageously with organic or inorganic arsenic compounds.

It is therefore an object of the present invention to provide the use of an organic or inorganic arsenic compound for preparing a medicament for the treatment of premenstrual syndrome.

Preferably, the medicament of the present invention is prepared in suitable dosage forms comprising an effective dose of at least one organic or inorganic arsenic compound and at least one pharmaceutically acceptable inert ingredient.

Typical examples of suitable inorganic arsenic compounds are: dibasic sodium arsenate ("The Merck Index", 12th ed., No. 8720), arsenic triiodide ("The Merck Index", 12th ed., No. 843), arsenic trioxide ("The Merck Index", 12th ed., No. 844) and potassium arsenite. Preferred examples are arsenic trioxide and potassium arsenite.

Typical examples of suitable organic arsenic compounds are: acetarsone ("The Merck Index", 12th ed., No. 49), arsacetin ("The Merck Index", 12th ed., No. 829), arsanilic acid ("The Merck Index", 12th ed., No. 830), arsenamide (or thiacetarsamide) ("The Merck Index", 12th ed., No. 831), arsenic acid ("The Merck Index", 12th ed., No. 833), arsenoacetic acid ("The Merck Index", 12th ed., No. 848), arsonoacetic acid ("The Merck Index", 12th ed., No. 850), arsphenamine ("The Merck Index", 12th ed., No. 851), cacodylic acid ("The Merck Index", 12th ed., No. 1640), carbarsone ("The Merck Index", 12th ed., No. 1830), dichlorophenarsine hydrochloride ("The Merck Index", 12th ed., No. 3121), diphetarsone ("The Merck index", 12th ed., No. 3394), ethanearsonic acid ("The Merck Index", 12th ed., No. 3768), glycarsamide ("The Merck Index", 12th ed., No. 4489), glycobiarsol ("The Merck Index", 12th ed., No. 4503), 3-methylarsacetin ("The Merck Index", 12th ed., No. 6102), neoarsphenamine ("The Merck Index", 12th ed., No. 5634), nitarsone ("The Merck Index", 12th ed., No. 6659), oxophenarsine hydrochloride ("The Merck Index", 12th ed., No. 7082), oxophenylarsine ("The Merck Index", 12th ed., No. 7083), roxarsone ("The Merck Index", 12th ed., No. 8430), sodium arsenylate ("The Merck Index", 12th ed., No. 8719), sodium arsphenamine ("The Merck Index, 12th ed., No. 8722), sulpharside ("The Merck Index", 12th ed., No. 9110), sulpharsphenamine ("The Merck Index", 12th ed., No. 9111), thiocarbamizine ("The Merck Index", 12th ed., No. 9458), thiocarbarsone ("The Merck Index", 12th ed., No. 9459), tryparsamide ("The Merck Index", 12th ed., No. 9925), melarsoprol ("The Merck Index", 12th ed., No. 5856), the potassium salt of melarsonyl (Trimelarsan™, Index Nominum, ed. 1987, p. 651) and melarsomine dihydrochloride (Immiticide™).

A preferred example of an organic arsenic compound is the disodium salt of arsenamide.

Examples of suitable dosage forms are tablets, capsules, coated tablets, granules, solutions and syrups for oral administration and sterile solutions for injection.

The dosage forms may also contain other conventional ingredients such as: preservatives, stabilizers, surfactants, buffers, salts for regulating the osmotic pressure, emulsifiers, sweeteners, colourings, flavourings and the like.

The amount of organic or inorganic arsenic compound in the dosage forms according to the present invention may vary within a wide range depending on known factors such as, for example, the severity of the syndrome to be treated, the patient's body weight, the route of administration selected and the number of daily administrations. However, the optimum amount may be determined easily and routinely by considering that the dosage will be such that it corresponds to an administration of 0.01-0.3 mg/kg/day, and even more preferably 0.05-0.2 mg/kg/day, of arsenic.

Preferably, the dosage will be such that it corresponds to an administration of about 0.05-0.01 mg/kg/day of arsenic in the case of injectable medicaments and, typically, about 0.05 mg/kg/day via the intravenous route and about 0.1 mg/kg/day via the intramuscular route. In the case of oral administration, the preferred dosage will be such that it corresponds to an administration of about 0.15-0.25 mg/kg/day.

The dosage forms according to the present invention may be prepared according to techniques that are well known to pharmaceutical chemists, including mixing, granulation, compression, dissolution, sterilization and the like.

The said dosage forms are preferably administered for 1-15 days, even more preferably 2-10 days, from the time at which the prodromic symptoms of PMS are exhibited.

The therapeutic effect is long-lasting and can thus be regarded as having a decisive impact on to the condition.

If the PMS has been present for a long time and/or if its symptoms are particularly severe, it is advantageous to follow up the treatment with a second course 40-120 days after the first course. The duration and dosage of the second course will be the same as or less than those of the first course.

The activity of the organic and inorganic arsenic compounds towards PMS was tested by means of the experiments described below.

### EXPERIMENTS

30 women between 19 and 41 years old, who had been suffering from premenstrual syndrome for at least 2 years, with symptoms ranging in intensity from mild to severe, were divided into three groups (of 10 women each) and given to the following treatments.

The expression "women suffering from serious PMS" is understood to refer to women who showed symptoms from the time of ovulation, with worsening of these symptoms during the luteinic phase and an improvement only after the end of menstruation. The mental symptoms encountered were: pronounced irritability and anxiety, outbursts of anger and panic attacks causing serious difficulties in relating to the external environment. The physical symptoms encountered were: very intense abdominal pains and headaches, which were resistant to common pharmacological treatments and were generally liable to lead to abstention from work or from other engagements; pain in the joints and difficulty in coordinating hand movements, with consequent problems in driving motor vehicles. Severe phenomena of water retention were also present, resulting, for example, in feet which could not fit into shoes and/or rings which could not be removed from the fingers.

The expression "women suffering from mild PMS" is understood to refer to women who show symptoms from the end of the luteinic phase until the start of the menstrual cycle. The mental symptoms such as anxiety and irritability were not present in every cycle, or appeared with differing intensity from one month to another. Abdominal pains and headaches were described as "tolerable" or subsided when common analgesics were taken. Other symptoms such as insomnia, disturbed appetite, mastalgia and pain in the joints were present periodically, while water retention in the form of a "swollen and heavy sensation" was constantly experienced. All of these symptoms had only a partial impact on the lifestyle of the women concerned.

0.33 ml/10 kg of an aqueous sterile 1% solution of disodium salt of arsenamide (equal to 0.66 mg/10 kg of arsenic) were administered intravenously to the first group for 5 days, in single daily doses.

Potassium arsenite (or Fowler's arsenical liquor) as an aqueous 0.5% solution, prepared under entirely sterile conditions in a laminar-flow fume cupboard containing two filters, from arsenic trioxide and potassium carbonate, was administered intramuscularly to the second group. The dosage used was 0.27 ml/10 kg/day, split into two intramuscular administrations (one every 12 hours), equal to a dose of 0.1 mg/kg/day of pure arsenic, for 5 days.

Arsenic trioxide (0.2 mg/kg/day, corresponding to 0.15 mg/kg/day of pure arsenic) split into 3 daily administrations (taken during meals) was administered orally for 8 days to the third group.

The 3 groups of patients were asked to complete a questionnaire on PMS during the first and second menstrual cycles following the treatment.

The results were as follows.

1st GROUP. At the start of the first menstrual cycle, 8 patients reported a pronounced improvement in symptoms, in particular mental and emotional symptoms, while in 3 cases, moderate levels of swollen sensation and abdominal pains were reported. Of the remaining 2 patients, one reported a moderate improvement in all the symptoms, while the other found no improvement.

At the start of the second menstrual cycle following the treatment, 8 patients reported a total remission of all the symptoms. Of the remaining 2 patients, one reported a gradual improvement in the symptoms, while the other reported a disappearance of the emotional symptoms and attenuation and shortening of the duration of the physical symptoms.

2nd GROUP. At the start of the first menstrual cycle following the treatment, 5 patients reported a marked improvement in all of the symptoms, 2 reported an improvement in symptoms such as insomnia, headache and mastalgia, although the emotional symptoms persisted, albeit attenuated. Another 2 patients reported less irritability before the cycle and an improvement in the symptoms related to water retention. The last woman of the group found no improvement.

At the second menstrual cycle, the abovementioned 5 patients continued to benefit from the improvements gained and asserted that they no longer suffered from the symptoms associated with PMS. The abovementioned first two patients reported a total remission of the physical symptoms and partial improvement in the emotional symptoms. The abovementioned second two patients announced a disappearance of tenderness of the breasts and mastalgia, accompanied by a definite improvement in mood in the premenstrual phase. Finally, the last woman reported a moderate attenuation of the physical symptoms and less irritability as the cycle approached.

3rd GROUP. At the start of the first menstrual cycle following the treatment, 4 patients showed a definite improvement in symptoms such as irritability, depression and outbursts of anger, and a marked attenuation in the physical symptoms, especially those associated with the digestive system. Another 3 patients reported a complete remission of the emotional symptoms and a moderate improvement in the physical symptoms, while 2 patients resported an attenuation of the physical symptoms but persistence of the emotional symptoms. The last woman reported that she had not found any improvement in the physical symptoms and had experienced a worsening of the emotional symptoms.

At the start of the second menstrual cycle after the treatment, 4 patients reported a strong attenuation not only in the physical symptoms but also in the mental and emotional symptoms. 3 patients reported a gradual improvement in the physical symptoms. 2 patients reported a partial improvement in the physical and emotional symptoms, while the last woman continued to show no appreciable change.

From the results obtained, it can be noted that the intravenous administration of the sodium salt of arsenamide gave the best therapeutic results, while the intramuscular administration of potassium arsenite showed good pharmacological activity, with fewer but equally long-lasting therapeutic successes. The oral administration of arsenic trioxide also gave satisfactory results, although not to the extent of the treatments by injection.

The use of the disodium salt of arsenamide did not modify the transaminase titre, either during or after the treatment.

However, the use of potassium arsenite produced transitory variations in the transaminases, of the order of 100-200%. However, the values returned to normal 4-6 days after the end of the treatment.

For the women who showed only partial or no improvements, a second treatment course 40 days after the first was envisaged, preferably using the sodium salt of arsenamide intravenously.

## Claims

1. Use of an organic or inorganic arsenic compound for preparing a medicament for the treatment of premenstrual symdrome.

2. Use of an arsenic compound according to Claim 1, **characterized in that** the said inorganic compound is chosen from the group comprising arsenic trioxide, dibasic sodium arsenate, potassium arsenite and arsenic triiodide.

3. Use of an arsenic compound according to Claim 2, **characterized in that** the said inorganic compound is arsenic trioxide.

4. Use of an arsenic compound according to Claim 1, **characterized in that** the said organic compound is chosen from the group comprising acetarsone, arsacetin, arsanilic acid, arsenamide, arsenic acid, arsenoacetic acid, arsonoacetic acid, arsphenamine, cacodylic acid, carbarsone, dichlorophenarsine hydrochloride, diphetarsone, ethanearsonic acid, glycarsamide, glycobiarsol, 3-methylarsacetin, neoarsphenamine, nitarsone, oxophenarsine hydrochloride, oxophenylarsine, roxarsone, sodium arsenylate, sodium arsphenamine,sulpharside,sulpharsphenamine, thiocarbamizine, thiocarbarsone, tryparsamide, melarsoprol, the potassium salt of melarsonyl and melarsomine dihydrochloride

5. Use of an arsenic compound according to Claim 4, **characterized in that** the said organic compound is the sodium salt of arsenamide.

6. Use of an arsenic compound according to any one of Claims 1 to 5, **characterized in that** the said medicament is to be administered orally or by injection.

## Patentansprüche

1. Verwendung einer organischen oder anorganischen Arsenverbindung zur Herstellung eines Medikaments zur Behandlung des prämenstruellen Syndroms.

2. Verwendung einer Arsenverbindung nach Anspruch 1, **dadurch gekennzeichnet, dass** die anorganische Verbindung ausgewählt ist unter Arsentrioxid, dibasischem Natriumarsenat, Kaliumarsenit und Arsentriiodid.

3. Verwendung einer Arsenverbindung nach Anspruch 2, **dadurch gekennzeichnet, dass** es sich bei der anorganischen Verbindung um Arsentrioxid handelt.

4. Verwendung einer Arsenverbindung nach Anspruch 1, **dadurch gekennzeichnet, dass** die organische Verbindung ausgewählt ist unter Acetarson, Arsacetin, Arsanilsäure, Arsenamid, Arsensäure, Arsenoessigsäure, Arsonoessigsäure, Arsphenamin, Kakodylsäure, Carbarson, Dichlorphenarsinhydrochlorid, Diphetarson, Ethanarsonsäure, Glycarsamid, Glycobiarsol, 3-Methylarsacetin, Neoarsphenamin, Nitarson, Oxophenarsinhydrochlorid, Oxophenylarsin, Roxarson, Natrium-arsenylat, Natrium-arsphenamin, Solpharsid, Solpharsphenamin, Thiocarbamizin, Thiocarbarson, Tryparsamid, Melarsoprol, das Kaliumsalz von Melarsonyl und Melarsomindihydrochlorid.

5. Verwendung einer Arsenverbindung nach Anspruch 4, **dadurch gekennzeichnet, dass** es sich bei der organischen Verbindung um das Natriumsalz von Arsenamid handelt.

6. Verwendung einer Arsenverbindung nach einem der Ansprüche 1 bis 5, **dadurch gekennzeichnet, dass** das erwähnte Medikament oral oder durch Injektion zu verabreichen ist.

## Revendications

1. Utilisation d'un composé organique ou inorganique de l'arsenic pour préparer un médicament destiné au traitement du syndrome prémenstruel.

2. Utilisation d'un composé de l'arsenic selon la revendication 1, **caractérisée en ce que** ledit composé inorganique est choisi dans le groupe comprenant le trioxyde d'arsenic, l'arséniate de sodium dibasique, l'arsénite de potassium et le triiodure d'arsenic.

3. Utilisation d'un composé de l'arsenic selon la revendication 2, **caractérisée en ce que** ledit composé inorganique est le trioxyde d'arsenic.

4. Utilisation d'un composé de l'arsenic selon la revendication 1, **caractérisée en ce que** ledit composé organique est choisi dans le groupe comprenant l'acétarsone, l'arsacétine, l'acide arsanilique, l'arsénamide, l'acide arsénique, l'acide arsénoacétique, l'acide arsonoacétique, l'arsphénamino, l'acide cacodylique, la carbarsone, le chlorhydrate de dichlorophénarsine, la diphétarsone, l'acide éthanearsonique, le glycarsamide, le glycobiarsol, la 3-méthylarsacétine, la néoarsphénamine, la nitarsone, le chlorhydrate d'oxophénarsine, l'oxophénylarsine, la roxarsone, l'arsénylate de sodium, l'arsphénamine de sodium, le sulfarside, la sulfarsphénamine, la thiocarbamizine, la thiocarbasone, le tryparsamide, le mélarsoprol, le sel de potassium du mélarsonyl et le dichlorhydrate de mélarsomine.

5. Utilisation d'un composé de l'arsenic selon la revendication 4, **caractérisée en ce que** ledit composé organique est le sel de sodium de l'arsénamide.

6. Utilisation d'un composé de l'arsenic selon l'une quelconque des revendications 1 à 5, **caractérisée en ce que** ledit médicament doit être administré par voie orale ou par injection.
